# EUROPÄISCHE PATENTANMELDUNG

(11) **EP 4 632 372 A1**
(43) Veröffentlichungstag der Anmeldung: **15.10.2025**
(21) Anmeldenummer: 25168428.8
(22) Anmeldetag: 04.04.2025
(51) Int. Cl.: G01N 33/00, G01N 1/22

(54) **VERFAHREN UND VORRICHTUNG ZUR DETEKTION EINER GASFÖRMIGEN EMISSION AUS EINER OBERFLÄCHE**

(30) Priorität: 12.04.2024 DE 102024110375
(71) Anmelder: Fraunhofer-Gesellschaft zur Förderung der angewandten Forschung e.V., 80686 München (DE)
(72) Erfinder: Gottschall, Sebastian, 01187 Dresden (DE); Boye, André, 01156 Dresden (DE); Fuchs, Enrico, 01705 Freital (DE)
(74) Vertreter: Gottfried, Hans-Peter

(57) **Zusammenfassung**

Die Erfindung betrifft eine Vorrichtung und ein Verfahren zur Detektion einer gasförmigen Emission aus einer Oberfläche (4), wobei wenigstens eine Gassensoranordnung (16) zur Feststellung wenigstens eines aus einem filmischen Rückstand (2) auf der Oberfläche (4) eines Feststoffs oder der Oberfläche (4) eines Fluids (30) emittierten gasförmigen Stoffs umfasst ist. Nach der Erfindung ist die Gassensoranordnung (16) zur Ermittlung unterschiedlicher Signalstärken ausgeführt, sodass durch eine Signalstärkenauswertung des Sensorsignals eine Lokalisierung von Verschmutzungen erfolgen kann.

## Beschreibung

Die Erfindung betrifft ein Verfahren und eine Vorrichtung zur Detektion einer gasförmigen Emission aus einer Oberfläche, wobei wenigstens eine Gassensoranordnung zur Feststellung wenigstens eines aus einem filmischen Rückstand auf der Oberfläche eines Feststoffs oder der Oberfläche eines Fluids emittierten gasförmigen Stoffs umfasst ist.

Die bei der Herstellung technischer Bauteile oder Baugruppen verwendeten Bearbeitungshilfsstoffe (wie beispielsweise Kühlschmierstoffe, Öle, Fette, Strahlmittel, Konservierungsmittel, Korrosionsschutzmittel) verbleiben auf der Oberfläche der Teile und Baugruppen und können in nachfolgenden Prozessschritten (z. B. Lackieren, Kleben, Beschichten, Fügen) die Qualität mindern oder zu fehlerhaften Produkten führen. Um die Bearbeitungshilfsstoffe zu entfernen, werden teilweise Reinigungsprozesse zwischen den einzelnen Herstellungsprozessen durchgeführt. Durch schwankende Prozessgrößen in den Reinigungsprozessen (steigende Verschmutzung, sinkende Reinigungsmittelkonzentration) oder schwankendem Verschmutzungszustand der Teile (stärker haftender Schmutz oder dickere Schichten des Rückstands) kann das Erreichen der notwendigen Bauteilsauberkeit bei gleichzeitig effizienter Prozessführung (lange Standzeiten des Reinigungsbads, niedrige Temperaturen, kurze Reinigungszeiten) der Reinigungsprozesse nicht immer gewährleistet werden. Daher muss das Erreichen der für den Nachfolgeprozess ausreichenden Sauberkeit kontrolliert werden.

Die Verschmutzungen auf den Bauteilen werden in partikuläre Verschmutzungen (Späne, Abrieb) und filmische Rückstände oder Verschmutzungen unterteilt, da in unterschiedlichen Anwendungen verschiedene Stoffe stören. Als Beispiel soll die Herstellung einer Ölwanne eines Kraftfahrzeugs herangezogen werden. Dort stellt verbliebenes Ziehöl aus der Fertigung kein Problem dar, metallische Partikel hingegen können das Getriebe beschädigen.

Da es sich bei Reinigungsprozessen immer um Verdünnungsreihen handelt, bei denen die Verschmutzung bzw. der entsprechende Stoff auf ein tolerierbares Maß verdünnt wird, ist eine perfekte Sauberkeit der Teile und Oberflächen nicht erreichbar. Es verbleiben immer kleine Mengen an Verschmutzungen auf der Oberfläche. Die notwendige oder ausreichende Sauberkeit, d. h. wieviel Verschmutzung auf der Oberfläche verbleiben darf, wird durch die Anforderungen des nachfolgenden Prozesses definiert. Beispielsweise sind Lösemittel-basierte Lackierprozesse deutlich robuster gegenüber Verschmutzungen auf den Teilen, da das im Lack enthaltene Lösemittel diese ablöst und der Lack direkt auf der Teileoberfläche haften kann. Wasserbasierte Lacke sind im Gegensatz dazu deutlich empfindlicher gegenüber verschmutzen Oberflächen.

In einem weiteren Anwendungsbereich - der Reinigung von Maschinen und Anlagen sowie ganzer Produktionsbereiche - ist die Nachfrage nach Lösungen, welche Verschmutzungen lokalisieren und charakterisieren sehr hoch.

Aus der Druckschrift EP 3 950 159 A1 ist eine Sensoreinrichtung für ein Reinigungssystem, umfassend einen Verschmutzungssensor und eine Lichtquelle, bekannt. Nach den Absätzen [0011] bis [0013] ist der Verschmutzungssensor insbesondere als optischer Sensor, beispielsweise eine Kamera, ausgeführt und sucht die Umgebung nach verschmutzten Bereichen ab. Bevorzugt beruht die Funktion dieses optischen Sensors auf einem Lumineszenzeffekt. Dabei wird ein Lichtspektrum ausgesandt, das bei der erwartbaren Verschmutzung (z. B. Bakterienbelag) einen einfach detektierbaren Lumineszenzeffekt hervorruft. Alternativ dazu ist es auch möglich, ein Mittel auf die zu prüfende Oberfläche aufzutragen, das bei der vorgesehenen Beleuchtung in Zusammenwirken mit der zu entfernenden Verschmutzung einen Lumineszenzeffekt hervorruft. Die Wellenlänge der von der Reinigungsvorrichtung oder in dem Anwendungsraum emittierten Lichtstrahlung kann beispielsweise außerhalb des sichtbaren Spektrums liegen und zumindest teilweise das UV- oder das IR-Spektrum umfassen.

Durch die fehlende Selektivität des Verfahrens können partikuläre und filmische Verunreinigungen oft nicht unterschieden werden. Außerdem können Schwankungen im Umgebungslicht zum Verfälschen des Messsignals führen. Zusätzlich ist das Verfahren auf die Fluoreszenzreaktion der Verschmutzungen angewiesen (Silikonöle zum Beispiel fluoreszieren nicht). Ein Bestimmen der Verschmutzungsdicke bzw. der Bauteilsauberkeit ist bei wechselnden Verschmutzungen nicht möglich, da unterschiedliche Stoffe unterschiedlich stark fluoreszieren. Besteht das Bauteil, welches auf Verschmutzungen kontrolliert werden soll, aus einem fluoreszierenden Material (z. B. Kunststoff, elektrische Leiterplatten), kann die Verschmutzung nicht erkannt werden.

Die Oberflächenspannung einer Oberfläche gibt an, wie gut diese benetzbar ist. Gut bekannt ist die Ausformung eines Wassertropfens auf hydrophober Oberfläche (niedrige Oberflächenspannung), wobei eine Kugel gebildet wird. Auf einer hydrophilen Oberfläche (hohe Oberflächenspannung) hingegen läuft der Wassertropfen breit. Da die filmischen Rückstände (Öle, Fette usw.) einen großen Unterschied in ihrer Oberflächenspannung zum Material des Teils aufweisen (Metalle haben beispielsweise eine hohe Oberflächenspannung), können bereits sehr dünne Filme große Änderungen in der messbaren Oberflächenspannung auslösen. Da aber auch Rückstände aus den Reinigungsprozessen (z. B. Tenside wie Seife) auf der Oberfläche für eine gute Benetzbarkeit sorgen, bedeutet die Messung einer guten Benetzbarkeit (hohe Oberflächenspannung) nicht immer auch das Vorliegen einer sauberen Oberfläche. Außerdem können beispielweise eingetrocknete filmische Rückstände bzw. Verschmutzungsschichten durch Prozesse wie dem Plasmaaktivieren hohe Oberflächenspannungen aufweisen, aber trotzdem den Nachfolgeprozess stören (z. B. blättert eine Lackschicht ab, da sie nur auf der Verschmutzung gut haftet, nicht aber auf dem eigentlichen Teil). Außerdem kann mittels der Oberflächenspannungsmessung nicht zwischen verschiedenen Verschmutzungen unterschieden werden.

In der Folge wird bestenfalls mit zu hohem Aufwand gereinigt und es werden die Reinigungsbäder nur unvollständig ausgenutzt, in dem sie frühzeitig ausgetauscht oder mit erhöhten Temperaturen und/oder erhöhten Reinigungsmittelkonzentrationen eingesetzt werden. Der dadurch steigende Ressourcenaufwand bei der Produktion hebt die Produktionskosten und belastet die Umwelt. Werden ungenügend gereinigte Teile nicht erkannt, können Spätausfälle beim Endkunden hohe Folgekosten, nicht zuletzt auch für den Hersteller, verursachen. In hygienekritischen Bereichen können die Folgen noch wesentlich dramatischer sein (Stichwort "Lebensmittelsicherheit").

Die Druckschrift US 2015 / 0 098 084 A1 zeigt, insbesondere in Fig. 7 und den Absätzen [0432], [0434], [0455], [0458] - [0462], [0753], eine Vorrichtung zur Detektion einer gasförmigen Emission aus einer Oberfläche (Abs. [0458] - [0462]), wobei wenigstens eine Gassensoranordnung (z. B. ein IMFS Sensor, Fig. 7, Abs. [0753]) zur Feststellung wenigstens eines aus einem filmischen Rückstand auf der Oberfläche eines Feststoffs (Abs. [0434], [0455]) emittierten gasförmigen Stoffs (Abs. [0432]) umfasst ist.

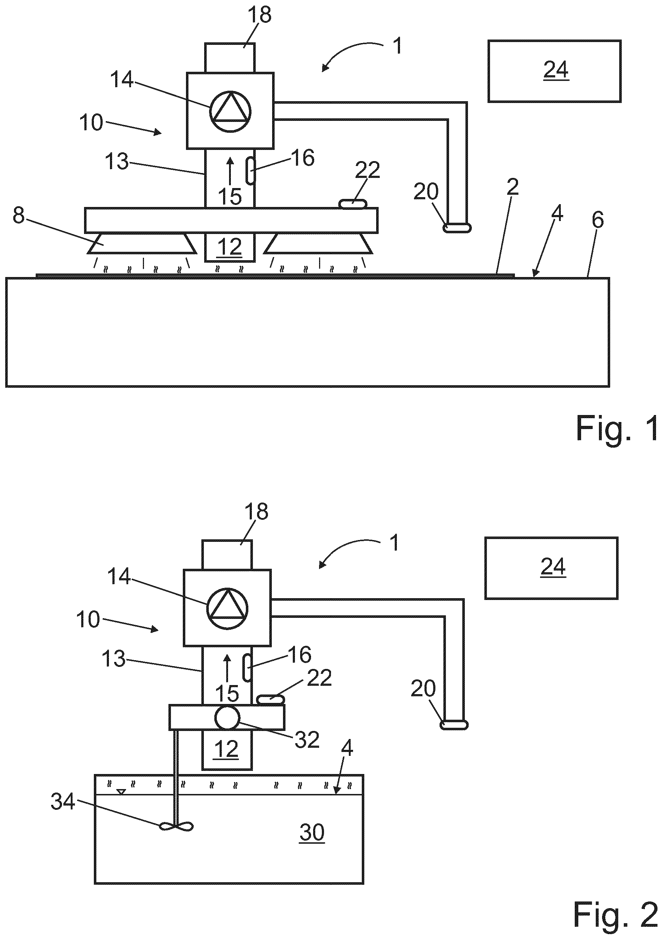

Die Druckschrift DE 100 14 335 A1 zeigt insbesondere in Fig. 1a, sowie in Sp. 1, Z. 21 - 28, Sp. 2, Z. 2 - 3 ebenfalls eine Vorrichtung zur Detektion einer gasförmigen Emission aus einer Oberfläche.

Die Lösungen aus dem Stand der Technik sind jedoch nicht geeignet, alle erforderlichen Angaben zu einer gasförmigen Emission zu ermitteln, insbesondere nicht den Ort ihres Auftretens.

Es ist daher Aufgabe der Erfindung, ein einfach handhabbares und sicheres Verfahren zur Detektion einer gasförmigen Emission aus einer Oberfläche sowie die dazu einzusetzende Vorrichtung anzubieten, um aus der gasförmigen Emission Rückschlüsse auf einen filmischen Rückstand, wie einer Verunreinigung oder Verschmutzung, auf einer Oberfläche oder auf den Zustand eines Fluids, insbesondere eines Wasch-, Reinigungs- oder Lösemittelfluids, über dort eingetragene, d. h. gelöste oder dispergierte, Rückstände oder Verschmutzungen, oder durch Verbrauch fehlende oder verminderte Gehalte an Zusatzstoffen festzustellen.

Die Aufgabe wird gelöst durch eine Vorrichtung zur Detektion einer gasförmigen Emission aus einer Oberfläche. Dabei ist wenigstens eine Gassensoranordnung zur Feststellung wenigstens eines aus einem filmischen Rückstand auf der Oberfläche, die eine Feststoffoberfläche darstellt, oder der Oberfläche eines Fluids, die eine Fluidoberfläche darstellt, emittierten gasförmigen Stoffs umfasst. Vorzugsweise ist auch eine zumindest mit der Gassensoranordnung zur Übertragung von Sensordaten verbundene Auswerteeinrichtung vorgesehen. Die Detektion des gasförmigen Stoffs lässt den Rückschluss auf das Vorhandensein oder die Art des filmischen Rückstands, beispielsweise einer Verschmutzung, zu.

Erfindungsgemäß ist die Gassensoranordnung zur Ermittlung unterschiedlicher Signalstärken ausgeführt, sodass durch eine Signalstärkenauswertung des Sensorsignals eine Lokalisierung von Verschmutzungen erfolgen kann. Dazu werden beispielsweise unterschiedliche Signalstärken erfasst und entsprechend signalisiert und/oder ausgewertet. Durch eine Signalstärkenauswertung des Sensorsignals kann eine Lokalisierung von Verschmutzungen erfolgen.

Im Falle der Nutzung der Erfindung als Handmessgerät, kann der Benutzer damit Verschmutzungsherde und Biofilme ausfindig machen, indem ihm anhand der Signalstärke signalisiert wird, in welcher Richtung der Verschmutzungsherd zu vermuten ist. Beim Schwenken und Bewegen des Handmessgeräts, vorzugsweise parallel zu Oberfläche, ist der Verschmutzungsherd in der Richtung zu vermuten, in der die Signalstärke höher wird. Bei linearer Bewegung im Raum wird das Signal umso stärker, je geringer die Entfernung zum Verschmutzungsherd ist.

Nach einer vorteilhaften Ausführungsform ist es vorgesehen, drei Gassensoreinrichtungen in der Weise zueinander beabstandet anzuordnen, dass aus den Messwerten von wenigstens drei der Gassensoreinrichtungen eine Positionsbestimmung der Emissionsquelle nach dem Prinzip der Triangulation möglich wird. Triangulation ist eine geometrische Methode der optischen Abstandsmessung durch genaue Winkelmessung innerhalb von Dreiecken. Die Berechnung erfolgt mittels trigonometrischer Funktionen. Dadurch wird es möglich von zwei Punkten, deren Abstand bekannt ist, Winkelmessungen zu beliebig anderen Punkten im Raum erfolgen, um deren Lage eindeutig zu bezeichnen. Sind drei Punkte bekannt, sind keine Winkelmessungen notwendig.

Soweit die Oberfläche die eines Wasch-, Reinigungs- oder Lösemittelfluids ist, kann über die Detektion des gasförmigen Stoffs auf dort eingetragene, d. h. gelöste oder dispergierte, Rückstände und damit den Grad der Verunreinigung des Fluids geschlossen werden. Außer diesem Effekt kann aber auch umgekehrt der Gehalt an bestimmten Zusatzstoffen im Fluid überwacht und damit der Verbrauch eines Wasch-, Reinigungs- oder Lösemittelfluids über fehlende oder verminderte Gehalte an Zusatzstoffen, festgestellt werden, was über die Detektion des gasförmigen Stoffs über der Oberfläche erfolgt.

Es hat sich als vorteilhaft erwiesen, wenn die wenigstens eine Gassensoranordnung als ein Sensor zur Überwachung eines Bereichs über der Oberfläche zur Feststellung des Vorhandenseins eines bestimmten gasförmigen Stoffs, wie Kohlendioxid CO₂ oder Wasserstoff H₂, ausgeführt ist. Die Luft im Bereich über der Oberfläche kann dabei über einem Bauteil oder über einem Fluid überwacht werden. Alternativ ist eine Kombination mehrerer unterschiedlicher Sensoren, insbesondere Sensoren für niedermolekulare, bis hochmolekulare Stoffe, vorgesehen, die unmittelbar über der Oberfläche oder beabstandet von der Oberfläche angeordnet sind.

Bei der Überwachung des Bereichs über der Oberfläche durch Analyse der dort anliegenden Luft kann die Emission von einem beliebigen Ort aus erfolgen. Eine räumliche Zuordnung ist zwar nicht oder kaum möglich, jedoch kann durch den Abstand der Sensoranordnung zur Oberfläche eine große Oberfläche auf einfache Weise überwacht werden. Dies ist insbesondere bei einem Fluid ausreichend, das über die gesamte Oberfläche hinweg eine im Wesentlichen gleichmäßige Konzentration und damit Ausgasung aufweist. Dabei kann die Luft im Kopfbereich eines Reinigungsbadbehälters überwacht werden.

Durch eine gezielte Mehrfachanordnung der Sensoren kann über eine Intensitätsüberwachung, eine Kombination von Sensorsignalen von mehreren Gassensoranordnungen oder mehreren Sensoren, die zu einer Gassensoranordnung zusammengefasst sind, die auch die vorhandene Intensität des gasförmigen Stoffs ermitteln, eine Lokalisierung der Geruchsquelle, d. h. den Ort der Emission, und somit eine Bestimmung des Verschmutzungsortes erfolgen. Zusätzlich kann auch eine Luftströmung im Messbereich Berücksichtigung finden und bei der Ermittlung des Orts der Emission korrigierend einbezogen werden.

Nach einer bevorzugten Ausführungsform ist eine Aktivierungseinrichtung zur Aktivierung des Rückstands und zur erhöhten Emission des wenigstens einen gasförmigen Stoffs umfasst. Nach einer ersten Alternative ist die Aktivierungseinrichtung als Heizeinrichtung zum zumindest lokalen Erwärmen der Oberfläche ausgeführt. Es hat sich dabei als vorteilhaft erwiesen, wenn die Heizeinrichtung zur Abgabe von Infrarotstrahlen, Mikrowellenstrahlung, UV-Strahlung oder/und zur Erzeugung einer elektrischen Aufladung ausgeführt ist.

Nach einer zweiten Alternative ist die Aktivierungseinrichtung für eine mechanische Wirkung ausgebildet. Stellt der Rückstand eine feste filmische Schicht dar, so trägt die Aktivierungseinrichtung Reibkräfte in den Rückstand ein und erzeugt dadurch eine erhöhte Emission des zu detektierenden gasförmigen Stoffs, beispielsweise durch Störung des Zusammenhalts der bereits ausgegasten Oberfläche oder einer ausgebildeten Kruste des Rückstands. Die Kruste wird durch die Reibkräfte zerkratzt und ermöglicht den ungehinderten Austritt des gasförmigen Stoffs aus darunterliegenden Schichten. Die mechanische Aktivierung kann auch durch eine Einrichtung erfolgen, die Ultraschall erzeugt und auf die filmische Schicht einwirken lässt. Der Ultraschall wird zumeist über ein Fluid von der Ultraschallquelle auf die filmische Schicht übertragen. Darüber hinaus kann das Fluid, dessen Oberfläche überwacht werden soll, mit Ultraschall beaufschlagt werden, um so ein verstärktes Austreten der zu detektierenden Gase zu erreichen.

Sofern die Detektion des gasförmigen Stoffs über der Oberfläche eines Fluids erfolgen soll, trägt die Aktivierungseinrichtung Scherkräfte in das Fluid ein, durchmischt die Schichten des Fluids und fördert das Ausgasen gelöster Stoffe über die Oberfläche. Eine solche Aktivierungseinrichtung ist beispielsweise als Rührer ausgeführt. Wird das Fluid gerührt, treten Gase verstärkt aus und können an der Oberfläche oder im Luftraum über der Oberfläche einfacher, schneller und genauer bzw. mit einer einfacheren, weniger sensiblen Gassensoranordnung detektiert werden.

Eine weitere vorteilhafte Ausführungsform umfasst eine Absaugeinrichtung zum Absaugen von Luft von der Oberfläche bzw. dem Bereich oberhalb der Oberfläche, beispielsweise dem Luftraum über der Oberfläche. Dies dient der Probennahme, indem die das zu detektierende Gas, den gasförmigen Stoff, enthaltende Luft über der beprobten Oberfläche abgesaugt und als Probe der Analyse zugeführt wird. Eine bevorzugte Ausführung mit einer Absaugeinrichtung umfasst eine Luftleiteinrichtung, wie z. B. eine Düse, sodass der Luftstrom gezielt aus einem eng begrenzten Bereich der Oberfläche zu der wenigstens einen Gassensoranordnung geführt wird und nur die aus einem begrenzten Bereich emittierten gasförmigen Stoffe festgestellt werden. Damit kann ein genaueres, örtlich aufgelöstes Bild über die Verschmutzung gewonnen werden und ein nachfolgender Reinigungsprozess gezielter erfolgen. Die Absaugeinrichtung kann auch genutzt werden, um die Gassensoranordnung gezielt anzublasen und so die Sensibilität der Erkennung auf einfache Weise zu erhöhen. Dazu ist die Luftleiteinrichtung jeweils entsprechend ausgeführt, wobei dem Fachmann verschiedene Möglichkeiten zur Verfügung stehen, aus denen er entsprechend seines Fachwissen auswählt.

Eine weitere vorteilhafte Ausführungsform umfasst wenigstens einen Oberflächenzustandssensor zur Ermittlung weiterer Parameter und Zustandsgrößen der Oberfläche, z. B. Material, Rauheit, Temperatur. Insbesondere die Temperatur beeinflusst die Ausgasung aus dem filmischen Rückstand wesentlich und wird daher vorzugsweise mit erfasst. Mit der Ermittlung weiterer Parameter und Zustandsgrößen der Oberfläche können zumindest eine Zusammensetzung und Eigenschaften verschiedener filmischer Rückstände unterschieden werden und insbesondere dem Umstand Rechnung getragen werden, dass bestimmte Parameter und Zustandsgrößen einerseits Art und Umfang der Verschmutzung und andererseits die notwendigen Reinigungsprozesse beeinflussen. Soweit die Oberfläche die eines Fluids ist, kommen andere Parameter und Zustandsgrößen zur zusätzlichen Erfassung in Betracht, insbesondere rheologische Eigenschaften.

Außerdem hat es sich gezeigt, dass sich Vorteile ergeben, wenn wenigstens ein Luftzustandssensor für die Ermittlung weiterer Eigenschaften der die Oberfläche umgebenden Luft, z. B. Temperatur, Luftfeuchte und/oder Druck, umfasst ist. Die Eigenschaften der Luft haben vor allem Einfluss auf die Abgabe der zu detektierenden Gase aus dem filmischen Rückstand sowie auf die Wechselwirkung mit der Luft in der Zeit zwischen Abgabe und Analyse, die das Analyseergebnis beeinflussen können. Die Wechselwirkungen und Einflüsse werden vorzugsweise bei der Auswertung der Sensordaten aller eingesetzten Sensoren in einer Auswerteeinrichtung berücksichtigt.

Als besonders vorteilhaft hat sich eine Einrichtung zur Aufkonzentration des emittierten gasförmigen Stoffs erwiesen, mit der genauere Ergebnisse der nachfolgenden Analyse mittels der Gassensoranordnung und eine erhöhte Sensibilität erreicht werden können. Somit können geringe, an sich nicht oder nur mit erhöhtem Aufwand detektierbare Konzentrationen überhaupt oder mit geringerem Aufwand, z. B. unter Verwendung einer einfacheren Gassensoranordnung, erfasst werden. Die Aufkonzentration kann auf einfache Weise bereits dadurch erfolgen, dass die Gassensoranordnung gezielt mit der von der Oberfläche abgesaugten Luft angeblasen wird. Verschiedene weitere Möglichkeiten stehen dem Fachmann zur Verfügung, aus denen er entsprechend seines Fachwissen auswählt.

Neben der Erfassung der Eigenschaften der Oberfläche und der umgebenden Luft hat sich die Ermittlung weiterer Größen als vorteilhaft erwiesen. Deshalb ist vorzugsweise wenigstens eine Einrichtung zur Erfassung von mechanischen Eigenschaften des Bauteils, zu dem die Oberfläche gehört, z. B. Geometrie, Größe und Form, vorgesehen. Sofern die Oberfläche die des Fluids ist, kommen anstelle von festen Zustandsgrößen typische Eigenschaften eines Fluids in Betracht, insbesondere rheologische Eigenschaften. In beiden Fällen kann die Genauigkeit der Ermittlung der Zusammensetzung des filmischen Rückstands, im Besonderen des Sauberkeitszustands, oder des Fluids, hier dessen Verunreinigung oder der Verbrauch von für den Reinigungserfolg notwendigen Inhaltsstoffen, erhöht werden.

Nach verschiedenen alternativen Ausführungsformen ist die Vorrichtung ausgeführt als Labormessgerät zur stationären Prüfung, als Handmessgerät zur mobilen Prüfung oder fest installiert in einer Fertigungsstrecke zur inline Messung, wo zudem eine Anbindung an eine Automatisierungstechnik mit besonderen Vorteilen verbunden ist. Dem Labormessgerät werden üblicherweise Muster zugeführt und im Labor die entsprechenden Gasanalysen durchgeführt. Das Handmessgerät zur mobilen Prüfung wird zu der zu prüfenden Oberfläche gebracht, um dort die gewünschten Analysen durchzuführen. Dies kann beispielsweise erfolgen, um in einer Anlage das Reinigungserfordernis oder den Reinigungserfolg festzustellen. Zur inline Messung erfolgt eine Festinstallation der Messtechnik, sodass sich vorbeibewegende Teile geprüft werden können. Ein solcher Einsatz ist beispielsweise zwischen verschiedenen Fertigungsstufen denkbar, die eine Zwischenreinigung erfordern. Im praktischen Einsatz kann ein solches Messsystem passiv an der Produktionslinie (also zwischen Reinigung und dem nächsten Fertigungsschritt) montiert sein. Die vorbeiziehenden Teile beeinflussen das Messsignal, was zum gewünschten Messergebnis führt.

Insbesondere bei einem Handmessgerät zur mobilen Prüfung hat es sich als vorteilhaft erwiesen, wenn die Gassensoranordnung zur Ermittlung unterschiedlicher Signalstärken ausgeführt ist, beispielsweise indem unterschiedliche Signalstärken erfasst und entsprechend signalisiert und/oder ausgewertet werden. Durch eine Signalstärkenauswertung des Sensorsignals kann eine Lokalisierung von Verschmutzungen erfolgen.

Im Falle der Nutzung der Erfindung als Handmessgerät, kann der Benutzer damit Verschmutzungsherde und Biofilme ausfindig machen, indem ihm anhand der Signalstärke signalisiert wird, in welcher Richtung der Verschmutzungsherd zu vermuten ist. Beim Schwenken und Bewegen des Handmessgeräts, vorzugsweise parallel zu Oberfläche, ist der Verschmutzungsherd in der Richtung zu vermuten, in der die Signalstärke höher wird. Bei linearer Bewegung im Raum wird das Signal umso stärker, je geringer die Entfernung zum Verschmutzungsherd ist.

Die Aufgabe der Erfindung wird ebenfalls gelöst durch ein Verfahren zur Detektion einer gasförmigen Emission aus einer Oberfläche, ein aus einem filmischen Rückstand auf der Oberfläche eines Feststoffs oder der Oberfläche eines Fluids emittierter gasförmiger Stoff, mit dem Ziel der Feststellung des filmischen Rückstands auf der Feststoffoberfläche oder des Zustands des Fluids unter der Oberfläche. Dabei wird wenigstens ein aus der Schicht des Rückstands emittierter gasförmiger Stoff festgestellt, wenn es sich um eine Schicht auf einem Feststoff, insbesondere einem Bauteil oder der Oberfläche einer Anlage, handelt. Erfolgt hingegen die Emission aus der Oberfläche eines Fluids, werden dessen Inhaltsstoffe, zumindest qualitativ relevante Anteile, quantitativ ermittelt. Die relevanten Anteile des Fluids können einerseits eingetragene Stoffe, z. B. im Zuge von Reinigungsvorgängen abgelöste und in das Fluid eingetragene Stoffe sein. Andererseits kann über das vorgeschlagene Verfahren festgestellt werden, ob essenzielle Komponenten des Fluids, z. B. Zusatzstoffe in einem Reinigungsbad, in ausreichender Konzentration vorhanden oder bereits verbraucht sind.

Nach der Erfindung erfolgt eine Lokalisierung des filmischen Rückstands auf der Oberfläche mittels Signalstärkenauswertung und/oder durch Auswertung von Messwerten von mehr als drei voneinander beabstandeten Gassensoranordnungen.

Zudem erfolgt nach der Detektion durch die Gassensoranordnung und aller anderen Sensoren vorzugsweise auch eine Übertragung der Sensordaten an eine Auswerteeinrichtung, um das Analyseergebnis entsprechend auszuwerten. Das Ergebnis der Auswertung kann zumindest lokal angezeigt oder zur Anzeige übertragen werden. Das Analyseergebnis kann aber auch in einer Auswerte- bzw. Steuerungseinrichtung verarbeitet werden und automatisierte Handlungen auslösen, wie z. B. eine Änderung der Reinigungsprozedur oder eine Ausschleusung von Teilen zur erneuten Reinigung.

Nach einer vorteilhaften Ausgestaltung des Verfahrens sind eine Überwachung der Qualität der Raumluft und/oder wenigstens eines bestimmten gasförmigen Stoffs wie z. B. CO₂ oder H₂ vorgesehen. Vorzugsweise wird zur Probennahme Luft bzw. das dort vorhandene Gas von der Oberfläche abgesaugt. Diese Ausgestaltung des Verfahrens wird besonders dort vorteilhaft angewendet, wo es um die Überwachung eines Fluids und die Emission gasförmiger Stoffe über die Oberfläche des Fluids geht. Hier kann die Raumluft in einem Kopfraum eines Behälters, der das Fluid, beispielsweise ein Reinigungsfluid, enthält, überwacht werden. In beiden Fällen kann die Überwachung alternativ oder zusätzlich im Umfang insoweit beschränkt werden, dass die Feststellung wenigstens eines bestimmten gasförmigen Stoffs erfolgt.

Sollen nur bestimmte Bereiche eines Bauteils auf Sauberkeit überprüft werden (z. B. Funktionsflächen zum Verkleben) können Mittel zum gezielten Absaugen dieser Bereiche genutzt werden. Das Absaugen erfolgt demnach vorteilhafterweise über eine Luftleiteinrichtung, wie z. B. eine Düse oder einen Adapter, sodass der Luftstrom gezielt zu der wenigstens einen Gassensoranordnung geführt wird und nur die aus einem begrenzten Bereich emittierten gasförmigen Stoffe festgestellt werden.

Das Absaugen kann ebenso über der Oberfläche eines Fluids erfolgen. In dem Fall bestehen die Vorteile darin, dass eine verlustarme Aufnahme der emittierten Stoffe erfolgen kann. Das zielgerichtete Absaugen verhindert beispielsweise ein Verwehen von gasförmigen Stoffen, die dann in verminderter Konzentration zu der Gassensoranordnung gelangen würden.

Als vorteilhaft hat sich weiterhin eine Aktivierung der Oberfläche zur erhöhten Emission des wenigstens einen gasförmigen Stoffs erwiesen, sodass dieser leichter zu detektieren ist, wobei die Aktivierung nach einer ersten Alternative durch zumindest lokale Erwärmung der Oberfläche und des darauf abgelagerten Rückstands erfolgt. Zur Erwärmung sind dazu Infrarotstrahlen, Mikrowellenstrahlung, UV-Strahlung oder/und eine elektrische Aufladung vorgesehen.

Nach einer zweiten Alternative erfolgt die Aktivierung der Oberfläche und/oder des Rückstands durch mechanische Beeinflussung. Wenn es sich um ein Fluid handelt, besteht die mechanische Beeinflussung in einem Eintrag von Scherkräften in das Fluid unterhalb der Oberfläche. Insbesondere wird das Fluid gerührt, sodass das Ausgasen von Inhaltsstoffen forciert wird.

Bei einer Feststoffoberfläche erfolgt ein Eintrag von Reibkräften in den Rückstand, der in der Folge ebenfalls gasförmige Stoffe leichter bzw. in größerer Menge abgibt, die dann entsprechend einfacher detektiert werden können. Zusätzlich hat es sich als vorteilhaft erwiesen, wenn zur Probennahme Luft von der Oberfläche abgesaugt wird, um verlustfrei die emittierten Gase der Gassensoranordnung zuzuführen.

Es ist weiterhin vorgesehen, zur mechanischen Aktivierung Ultraschall einzusetzen. Dieser kann sowohl zur Aktivierung der Feststoffoberfläche und/oder des Rückstands als auch zur Aktivierung eines Fluids eingesetzt werden, sodass das Ausgasen von Inhaltsstoffen forciert wird.

Darüber hinaus hat es sich gezeigt, dass wenigstens eine Ermittlung weiterer Parameter und Zustandsgrößen der festen Oberfläche, z. B. Material, Rauheit, Temperatur, oder rheologischer Eigenschaften des Fluids vorteilhaft ist, sodass zumindest eine Zusammensetzung und Eigenschaften verschiedener filmischer Rückstände und deren Ausbildung bzw. unterschiedliche Fluide unterschieden werden können. Zusätzlich wird vorzugsweise wenigstens eine weitere Eigenschaft der die Oberfläche umgebenden Luft, z. B. Temperatur, Luftfeuchte, Druck, erfasst.

Eine weitere vorteilhafte Ausgestaltung des Verfahrens besteht darin, die emittierten gasförmigen Stoffe vor der Zuführung der Luft, die die Stoffe enthält, zum Sensor aufzukonzentrieren, um eine höhere Sensibilität der Analyse zu erreichen.

Um die Genauigkeit des erfindungsgemäßen Verfahrens darüber hinaus weiter zu erhöhen, wird wenigstens eine mechanische Eigenschaft des Bauteils, zu dem die Oberfläche gehört, z. B. Geometrie, Größe, Form, erfasst. Dadurch kann die Genauigkeit der Ermittlung des Sauberkeitszustands erhöht werden.

Werden entweder verschiedene Verfahren im Sinne der Erfindung oder das gleiche Verfahren mit unterschiedlicher Intensität (z. B. mit geänderter Amplitude oder Leistung) zur Extraktion der detektierbaren Bestandteile verwendet, können ebenfalls die Zusammensetzung, Dicke oder andere Eigenschaften, die den filmischen Rückstand charakterisieren, detektiert werden und detailliert Aufschluss über den Zustand der betreffenden Oberfläche gegeben werden. Entsprechendes trifft auf mechanische Eigenschaften des Fluids, wie deren rheologische Eigenschaften, zu.

Durch bestimmte Stoffzusätze oder die konkret ausgewählte Zusammensetzung der ohnehin eingesetzten Bearbeitungshilfsstoffe kann gezielt die Detektierbarkeit des später auf den Teilen verbleibenden filmischen Rückstands, auch bei zunächst nicht "riechenden" Stoffen, die zu wenige detektierbare Emissionen hervorrufen, ermöglicht oder verbessert werden. Dies erfolgt insbesondere mittels eines Zusatzes leicht flüchtiger, gut durch die Gassensoranordnung detektierbarer Stoffe.

Bei der Bewertung des Hygienezustands von Produktionsumgebungen kann die Sensorik bzw. die erfindungsgemäße Vorrichtung über die Messung eines Gradienten, insbesondere des Anstiegs des Signals zur Lokalisierung von Verschmutzungen genutzt (vergleichbar mit dem Einsatz eines Metalldetektors), aber genauso auch zur Bestimmung der Schmutzmenge sowie der Art (z. B. zur Detektion von Biofilmen) herangezogen werden. Bevorzugt erfolgt daher eine Messung des Anstiegs des Signals über den Weg, um bei der Bewegung Verschmutzungen auf der Oberfläche konkret lokalisieren zu können.

Bei einer alternativen Ausführungsform der Erfindung wird die Gassensoranordnung im luftgefüllten Kopfbereich des Reinigungsbadbehälters angebracht, da hier die Konzentration der flüchtigen, Verschmutzungen anzeigenden Verbindungen besonders hoch ist. Speziell werden hier mittels einer Gassensoranordnung für Fluide, insbesondere Waschbäder, die Verringerung enthaltener Additive und die Verschmutzung im Waschbad erfasst. Hier ist vor Beginn der Messung ein Luftaustausch, eine Frischluftbefüllung des Luftreservoirs, mit sich anschließender integraler Messung von Vorteil.

Die eingesetzte Reinigungsflüssigkeit wird in vielen Branchen wiederverwendet. Hierdurch kann es zu einer Verschleppung von Verunreinigungen kommen, weshalb auch der Verschmutzungsgrad des Reinigungsbads selbst großen Einfluss auf die erreichbare Sauberkeit der zu reinigenden Teile hat. Um den Verschmutzungsgrad zu bestimmen, fehlen bisher geeignete bzw. wirtschaftliche Technologien. Zusätzlich führt der verminderte Einsatz von Chemikalien zu einem erhöhten Wachstum an Mikroorganismen in den Reinigungsbädern, was eine weitere Kontaminationsquelle darstellt und zu einer Gefahr für die Bediener und die Produktion werden kann. Daher ist eine Überwachung des Reinigungsbades zusätzlich erforderlich.

Die Vorteile der vorliegenden Erfindung liegen nicht zuletzt darin begründet, dass Gassensoren und Raumluftsensoren sowie Sensorik für das Raumklima weitverbreitet und kostengünstig verfügbar sind. Wird in dem erfindungsgemäßen System einer oder mehrere dieser Sensoren eingesetzt, ist die komplette Lösung ebenfalls kostengünstiger realisierbar im Vergleich zu Lösungen nach dem Stand der Technik.

Durch die gute Inlinefähigkeit des erfindungsgemäßen Messverfahrens (Einsatz in der Produktionskette) kann eine kostengünstige und umfassende Kontrolle der Teile realisiert werden, die Ausschuss zu vermeiden und die Prozesse effizient zu führen hilft. Dies realisiert sich beispielsweise darin, dass ein zu früher Austausch von Reinigungsbädern vermieden wird.

Im Gegensatz zur Oberflächenspannungsmessung ist das Verfahren berührungslos und störungsfrei bzw. beeinflusst die zu betrachtenden Oberflächen nicht. Somit besteht eine geringe Kontaminationsgefahr durch den Sensor.

In der Fluoreszenzmesstechnik werden nicht fluoreszierende Verschmutzungen oft mit fluoreszierenden Farbstoffen (Tracern) versetzt. Diese sind aber im Vergleich zum Bearbeitungshilfsstoff sehr teuer und stellen eine zusätzliche Fehlerquelle und einen Störstoff für die nachfolgenden Prozesse dar. Im erfindungsgemäßen Verfahren kann hingegen durch gezielte Auswahl des am stärksten riechenden Stoffs eine gute Detektierbarkeit sichergestellt werden. Alternativ sind leicht flüchtige Zusatzstoffe möglich, die die nachfolgenden Prozesse nicht beeinflussen.

Eine technische Anwendung der Erfindung kann vorteilhaft in den folgenden Bereichen erfolgen:
1. Bauteilfertigung (mechanische oder elektrische Teile und Baugruppen): In Fertigungsprozessen, bei denen ein Stoff im Prozess absichtlich oder unabsichtlich auf die Teile gelangt, wirkt dieser später als Störstoff in einem nachfolgenden Prozess (z. B. Kleben, Lackieren, Fügen, Dichten) und muss in einer Zwischenreinigung effizient entfernt werden.
2. Medizintechnik: Die filmischen Rückstände begünstigen Keimwachstum oder direkt die Kontamination des Patienten oder dessen Umfeld.
3. Halbleiterbranche: Die filmischen Rückstände, die auf den in der Halbleiterfertigung verwendeten Bauteilen, Transportmitteln oder Werkzeugen verbleiben, dünsten im Reinraum aus und Kontaminieren den Reinraum.
4. Lebensmittelproduktion: Reinigung von Anlagentechnik zur Verbesserung von Hygiene (Verhinderung oder Beschränkung von Keimwachstum) und Sicherheit bei effektivem Ressourceneinsatz zur Reinigung.
5. Bioverfahrenstechnik: siehe Ziff. 4.
6. Pharmaindustrie: siehe Ziff. 4.

Anhand der Beschreibung von Ausführungsbeispielen und ihrer Darstellung in den zugehörigen Zeichnungen wird die Erfindung nachfolgend näher erläutert. Fig. 1 zeigt schematisch eine Ansicht einer Ausführungsform einer erfindungsgemäßen Vorrichtung 1 sowie einen filmischen Rückstand 2 auf einer Oberfläche 4 eines Bauteils 6.

Die Vorrichtung 1 umfasst eine Absaugeinrichtung 10, die einen aus dem filmischen Rückstand 2 abgegebenen gasförmigen Stoff von der Oberfläche 4 absaugt. Die Absaugeinrichtung 10 umfasst eine Saugpumpe 14, die einen Luftstrom 15 in einem Luftrohr 13 hervorruft. Das Luftrohr 13 reicht mit einer Luftleiteinrichtung, ausgeführt als eine Düse 12, an die Oberfläche 4 heran. Die von der Oberfläche 4 abgesaugte Luft, der Luftstrom 15, passiert eine Gassensoranordnung 16, mit der die Bestandteile des abgesaugten gasförmigen Stoffs ermittelt bzw. deren Vorhandensein festgestellt werden, und verlässt das Luftrohr 13 über einen Luftaustritt 18.

Zur Aktivierung des filmischen Rückstands 2 mit dem Ziel, eine größere Menge des gasförmigen Stoffs freizusetzen, dient eine Aktivierungseinrichtung, ausgeführt als Heizeinrichtung 8. Die Heizeinrichtung 8 strahlt Wärme auf den filmischen Rückstand 2, wobei die Wärmestrahlung durch kurze gerade Linien dargestellt ist, worauf sich der filmische Rückstand 2 erwärmt. Der erwärmte filmische Rückstand 2 strahlt seinerseits Wärme zurück, dargestellt durch kurze doppelte Wellenlinien, und gibt zugleich eine größere Menge des gasförmigen Stoffs ab, im Vergleich zum nicht aktivierten Zustand.

Weitere Messwerte werden durch einen Luftzustandssensor 22 ermittelt, der die Eigenschaften der Umgebungsluft, die das Bauteil 6 umgibt, ermittelt. Zusätzlich generiert ein Oberflächenzustandssensor 20 Messwerte, insbesondere werden mechanische Zustandswerte der Oberfläche 4 und/oder des Bauteils 6 ermittelt. Die Sensordaten der Gassensoranordnung 16, des Oberflächenzustandssensors 20 und des Luftzustandssensors 22 werden an eine Auswerteeinrichtung 24 übermittelt, was über nicht dargestellte Leitungen oder drahtlos erfolgen kann. In der Auswerteeinrichtung 24 werden die Ergebnisse aus der Gassensoranordnung 16 in Beziehung gesetzt zu den übrigen Messwerten, die der Oberflächenzustandssensor 20 und der Luftzustandssensor 22 beisteuern. Im Ergebnis können insbesondere Prozessparameter für einen Reinigungsvorgang abgeleitet werden.

Fig. 2 zeigt schematisch eine Ansicht einer Ausführungsform einer erfindungsgemäßen Vorrichtung 1 entsprechend Fig. 1, hier jedoch über einer Oberfläche 4 eines Fluids 30. Gegenüber der in Fig. 1 gezeigten Vorrichtung 1 ist eine Einrichtung 32 zur Aufkonzentration der über die Luftleiteinrichtung 12 eingezogenen gasförmigen Stoffe. Deren Emission über die Oberfläche 4 des Fluids 30 wird forciert durch einen Rührer 34, der als Einrichtung zur mechanischen Beeinflussung des Fluids dient und dort Scherkräfte einträgt.

### Bezugszeichenliste

- 1: Vorrichtung
- 2: filmischer Rückstand
- 4: Oberfläche
- 6: Bauteil
- 8: Aktivierungseinrichtung, Heizeinrichtung
- 10: Absaugeinrichtung
- 12: Luftleiteinrichtung, Düse
- 13: Luftrohr
- 14: Saugpumpe
- 15: Luftstrom
- 16: Gassensoranordnung
- 18: Luftaustritt
- 20: Oberflächenzustandssensor
- 22: Luftzustandssensor
- 24: Auswerteeinrichtung
- 30: Fluid
- 32: Einrichtung zur Aufkonzentration
- 34: Einrichtung zur mechanischen Beeinflussung, Rührer

## Patentansprüche

1. Vorrichtung zur Detektion einer gasförmigen Emission aus einer Oberfläche (4), wobei wenigstens eine Gassensoranordnung (16) zur Feststellung wenigstens eines aus einem filmischen Rückstand (2) auf der Oberfläche (4) eines Feststoffs oder der Oberfläche (4) eines Fluids (30) emittierten gasförmigen Stoffs umfasst ist, **dadurch gekennzeichnet, dass** die Gassensoranordnung (16) zur Ermittlung unterschiedlicher Signalstärken ausgeführt ist, sodass durch eine Signalstärkenauswertung des Sensorsignals eine Lokalisierung von Verschmutzungen erfolgen kann.

2. Vorrichtung nach Anspruch 1, wobei wenigstens drei Gassensoreinrichtungen (16) in der Weise zueinander beabstandet sind, dass aus deren Messwerten eine Positionsbestimmung der Emissionsquelle möglich wird.

3. Vorrichtung nach Anspruch 1 oder 2, wobei die wenigstens eine Gassensoranordnung (16) zur Feststellung des Vorhandenseins eines bestimmten gasförmigen Stoffs in einem Bereich über der Oberfläche (4) oder als eine Kombination mehrerer unterschiedlicher Sensoren ausgeführt ist.

4. Vorrichtung nach einem der Ansprüche 1 bis 3, wobei eine Aktivierungseinrichtung (8) zur Aktivierung des Rückstands (2) oder Fluids (30) umfasst ist, wobei die Aktivierungseinrichtung (8) zur erhöhten Emission des wenigstens einen gasförmigen Stoffs beiträgt, wobei nach einer ersten Ausführungsform die Aktivierungseinrichtung (8) als Heizeinrichtung zum zumindest lokalen Erwärmen oder als Einrichtung zur mechanischen Beeinflussung der Oberfläche (4) und/oder des Rückstands (2) ausgeführt ist, wobei nach einer zweiten Ausführungsform die Heizeinrichtung zur Abgabe von Infrarotstrahlen, Ultraschall, Mikrowellenstrahlung, UV-Strahlung oder/und zur Erzeugung einer elektrischen Aufladung ausgeführt ist, wobei nach einer dritten Ausführungsform die Einrichtung zur mechanischen Beeinflussung zum Eintrag von Scherkräften in das Fluid (30) unterhalb der Oberfläche (4) oder zum Eintrag von Reibkräften in den Rückstand (2) oder als Einrichtung zur Erzeugung von Ultraschall ausgeführt ist.

5. Vorrichtung nach einem der vorherigen Ansprüche, wobei zur Probennahme eine Absaugeinrichtung (10) zum Absaugen von Luft von der Oberfläche (4) umfasst ist.

6. Vorrichtung nach einem der vorherigen Ansprüche, wobei wenigstens ein Luftzustandssensor (22) für die Ermittlung weiterer Eigenschaften der die Oberfläche (4) umgebenden Luft umfasst ist.

7. Vorrichtung nach einem der vorherigen Ansprüche, wobei eine Einrichtung zur Aufkonzentration (32) des emittierten gasförmigen Stoffs umfasst ist.

8. Vorrichtung nach einem der vorherigen Ansprüche, ausgeführt als Labormessgerät zur stationären Prüfung, als Handmessgerät zur mobilen Prüfung oder zur inline Messung in einer Fertigungsstrecke mit Anbindung an eine Automatisierungstechnik.

9. Verfahren zur Detektion einer gasförmigen Emission aus einer Oberfläche (4), wobei wenigstens ein aus einem filmischen Rückstand (2) auf der Oberfläche (4) eines Feststoffs oder der Oberfläche (4) eines Fluids (30) emittierter gasförmiger Stoff festgestellt wird, **dadurch gekennzeichnet, dass** eine Lokalisierung des filmischen Rückstands (2) auf der Oberfläche (4) mittels Signalstärkenauswertung und/oder durch Auswertung von Messwerten von mehr als drei voneinander beabstandeten Gassensoranordnungen (16) erfolgt.

10. Verfahren nach Anspruch 9, wobei eine Überwachung der Qualität der Raumluft und/oder wenigstens eines bestimmten gasförmigen Stoffs erfolgen.

11. Verfahren nach Anspruch 9 oder 10, wobei die Oberfläche (4) zur erhöhten Emission des wenigstens einen gasförmigen Stoffs aktiviert wird, wobei die Aktivierung durch zumindest lokale Erwärmung der Oberfläche (4) oder durch mechanische Beeinflussung der Oberfläche (4) und/oder des Rückstands (2) erfolgt.

12. Verfahren nach Anspruch 11, wobei die Erwärmung durch Infrarotstrahlen, Mikrowellenstrahlung, UV-Strahlung oder/und eine elektrische Aufladung erfolgt.

13. Verfahren nach Anspruch 11 oder 12, wobei die mechanische Beeinflussung durch einen Eintrag von Scherkräften in das Fluid (30) unterhalb der Oberfläche (4) oder durch einen Eintrag von Reibkräften in den Rückstand (2) oder durch die Einwirkung von Ultraschall erfolgt.

14. Verfahren nach einem der Ansprüche 9 bis 13, wobei zur Probennahme Luft von der Oberfläche (4) abgesaugt wird, wobei die emittierten gasförmigen Stoffe aufkonzentriert werden.

15. Verfahren nach einem der Ansprüche 9 bis 14, wobei der Stoff, der den filmischen Rückstand (2) ausbildet, mit Stoffzusätzen versetzt wird oder Bearbeitungshilfsstoffe definiert zusammengesetzt werden, um eine Detektierbarkeit des später auf der Oberfläche (4) verbleibenden filmischen Rückstands (2) zu ermöglichen oder zu verbessern.
